(19) Europäisches Patentamt
European Patent Office
Office européen des brevets

(11) Publication number: **0 278 647 A2**

(12) # EUROPEAN PATENT APPLICATION

(21) Application number: 88300814.6

(51) Int. Cl.⁴: C12M 1/40

(22) Date of filing: 01.02.88

(30) Priority: 09.02.87 US 15353

(43) Date of publication of application: 17.08.88 Bulletin 88/33

(84) Designated Contracting States: DE FR GB NL

(71) Applicant: AMERICAN TELEPHONE AND TELEGRAPH COMPANY
550 Madison Avenue
New York, NY 10022(US)

(72) Inventor: Degani, Yinon
311B Crowells Road
Highland Park New Jersey 08904(US)
Inventor: Heller, Adam
7 Timberline Drive
Bridgewater New Jersey 08807(US)

(74) Representative: Johnston, Kenneth Graham et al
AT&T (UK) LTD. AT&T Intellectual Property Division 5 Mornington Road
Woodford Green Essex, IG8 OTU(GB)

(54) **Electronchemical processes involving enzymes.**

(57) The modification of enzymes through chemical attachment of an electronically active material such as a ferroceneamide or a ferroceneacetamide, or through chemical conversion of functional groups of an enzyme, such as conversion of enzyme tyrosine-amides to enzyme dihydroxyphenylalanine amides, allows previously unattainable direct interactions between the enzyme and an electrode. Thus, it is possible to drive chemical reactions involving the enzyme directly by interaction with an electrode, or conversely it is possible to monitor chemical reactions involving the enzyme directly at an electrode. This ability has consequences for chemical synthesis, for the assay of biochemicals, and for the local perturbation of the concentration of reducible or oxidizable biochemicals in biological systems by means of electrode processes involving the chemically-modified enzymes.

EP 0 278 647 A2

# ELECTROCHEMICAL PROCESSES INVOLVING ENZYMES

## Background of the Invention

### 1. Technical Field

This invention relates to biochemical reactions and in particular to biochemical reactions involving enzymes.

### 2. Art Background

Enzymes (i.e., macromolecules, for example protein containing macromolecules, that accelerate a chemical reaction) are used for a variety of processes. Redox enzymes are used in the electrochemical syntheses of biochemicals as well as in the assay and monitoring of biologically significant chemicals. In both applications, a fluid containing the enzyme interacts with an electrode. In reductive syntheses and assays, electrons are transferred from the electrode to the enzyme to induce a chemical reaction. In oxidative syntheses and assays, electrons are transferred from the enzyme to the electrode. Thus, not only are significant biochemical reactions driven electrochemically, but by monitoring the rate and/or potential of the electron transfer process, it is also possible to obtain a measure of reactant concentration, i.e., concentration of the material that is enzymatically oxidized or reduced.

The redox centers of enzymes do not interact directly with electrodes as a result of their involvement in accelerating a desired reaction. (Some interaction might occur due to extraneous processes such as oxidation or reduction of the enzyme unrelated to the enzymatically catalyzed reaction. These extraneous processes and the resulting current are independent of reactant concentration.) Usually modification of enzymes lead to their deactivation. For example, modification of carboxy groups in the enzyme bovine carboxypeptidase A, by N-ethyl-5-phenylisoxazolium-3′-sulfonate leads to its deactivation as reported by P. H. Petra in Biochemistry, 10, 3163 (1971). Possibly, because chemical modification appears to be an unproductive approach, to produce the desired charge transfer between the electrode and the enzyme, a low molecular weight reductant, oxidant, or redox couple is added to the electrochemical medium. The added species diffuses into the enzyme,exchanges charge, diffuses out of the enzyme, and reacts with the electrode. For example, for the enzyme glucose oxidase [E.C. (enzyme catalogue) 1.1.3.4], oxygen is introduced into the fluid, diffuses into the enzyme, is reduced by the glucose-reduced enzyme to hydrogen peroxide, which is electrochemically or chemically assayed. Similarly, in the production of pure L-amino acids from their mixtures with D-amino acids by oxidation with D-amino acid oxidase, oxygen, or an oxidant such as quinone carboxylic acid, is introduced to insure transfer of electrons from the enzyme to the oxidant, and from the oxidant to the electrode.

For many applications the introduction of these low molecular weight entities is not entirely desirable. The assay of biochemicals becomes dependent on the concentration of these added entities. Since this concentration is difficult to control, a concomitant inaccuracy is introduced into the assay. Thus, for example, when glucose is being assayed by oxidation with oxygen in the presence of glucose oxidase, the rate of production of $H_2O_2$ depends on the concentration of the dissolved oxygen. Since the current detected depends on the $H_2O_2$ concentration, this shifting limits the accuracy of the assay. Similarly, for the induction of chemical reactions, the introduction of a redox couple introduces impurities which for specialized applications are not acceptable. Therefore, for many significant applications, the use and/or presence of a separate redox couple is not desirable, and yet, without this couple the desired process does not occur.

## Summary of the Invention

Modification of an enzyme to interact directly with an electrode has been accomplished. This modification is achieved, for example, by chemically reacting the enzyme with a material having at least one functional group that chemically bonds to sites on the enzyme and at least one second group that acts as a redox couple. Alternatively, functional groups on the enzyme are converted to redox centers. Electron transfer occurs first from the chemically-active region of the enzyme to the introducted redox center and

then either from this center directly to the electrode or to other bound redox center or centers and then to the electrode. This enzyme modification is produced without unacceptable loss in enzymatic activity. A contemplated explanation is that the redox center is removed a sufficient distance from the intrinsic catalytic center or centers of the enzyme to prevent deactivation but is sufficiently close to them to allow transfer of electrons, possibly through processes such as tunnelling.

Enzymes so modified allow processes that involve direct charge transfer between the electrode and the enzyme. For example, glucose oxidase modified to form amide linkages with ferrocenyl or ferrocenyl-containing groups directly interacts wtih an electrode. Thus, the presence of glucose, a material that reduces glucose oxidase, is detected by monitoring the rate and/or potential of electrons passed directly from the reduced enzyme to the electrode. In general, this technique of adding a redox center at a position removed from the reversibly reducible/oxidizable center of the enzyme is effective in promoting interaction of an electrode directly with the enzyme and, thus, in the promoting of processes in which such direct interaction is desirable.

## Brief Description of the Drawing

The Figures are illustrative of resuilts obtainable with the invention.

## Detailed Description

The electrochemical applications involved in this invention are generally performed in a homogeneous or heterogeneous aqueous medium in the pH range between 4 and 9. Fluids other than those based on water generally induce deactivation of enzymes and, thus, are typically not desirable. Thus, generally in practice, reactants in an aqueous medium are oxidized or reduced in the presence of the modified enzyme, or in a heterogeneous system containing the modified enzyme immobilized on a solid such as a glass, a polymer, or a metal. (An aqueous-based fluid is any fluid containing at least 60 percent water and includes biological fluids such as blood.) An electrode is generally contacted with this fluid simply by immersion. As discussed, it is possible to attach the chemically modified enzyme to the conducting electrode and contact this electrode with the fluid. To complete the electrochemical process, a counter electrode that electrically interacts with the aqueous medium is employed. (A reference electrode is also commonly used, though its presence is not essential.) Again, the cell has at least two electrodes, e.g., metal electrodes, such as gold, platinum, or conductive carbon, or alternatively an electrode that is part of a semiconductor-based circuit. Electrical interaction is conveniently produced by simply immersing the electrodes and applying an appropriate potential. Typical counter electrodes are gold, platinum, or carbon. Convenient reference electrodes include $Hg/Hg_2Cl_2$ in chloride ion-containing solutions and Ag/AgCl in chloride ion-containing solutions.

The reactants, e.g., the material being tested, or the material reacting to form the desired product(s), are introduced into the aqueous mixture involved in the electrochemical cell. It is generally advantageous that these materials be introduced by solvating them. It is possible to adjust the PH of the aqueous medium to advantageously effect this dissolution. However, it is generally desirable to maintain a pH in the range 4 to 9 so that the enzyme is not adversely affected. For example in the case of interactions involving glucose, the glucose is dissolved in an aqueous medium having a pH of approximately 7.2. Methods of maintaining an appropriate pH by means of conventional or biological buffers are useful.

Enzymes are generally soluble in an aqueous medium. The modifications involved in the invention do not appreciably modify this solubility. Thus, the modified enzyme, if not attached to an electrode, is also advantageously introduced into the aqueous medium by addition of further solvent. The electrical conductivity, both anionic and cationic, necessary for an electrochemical process, is supplied by all the dissolved ions including the enzyme. At least part of the Faradaic electrode reactions involve charge transfer to or from the enzyme. Further introduction of redox couples, oxidants, or reductants other than the biochemicals involved in the reactions, is not required. (However, such introduction is not precluded.)

The enzyme to be employed is modified by the introduction of one or more redox centers, e.g., by the introduction of a reversibly oxidizable/reducible species. Exemplary redox centers are ferrocene derivatives, indophenols, hydroquinones, aromatic hydroxyamines, alkylviologens, or other redox entities. This introduction of redox centres into the enzyme is advantageously accomplished by reacting the enzyme with an

entity having a functional group that reacts with the enzyme and having a second functional group that acts as a redox center. For example, in the case of glucose oxidase, a suitable modification occurs through the formation of an amide linkage with either ferrocenecarboxylic acid or ferrocenylacetic acid. A modification by one such reaction sequence is represented for ferrocenecarboxylic acid by the formulae:

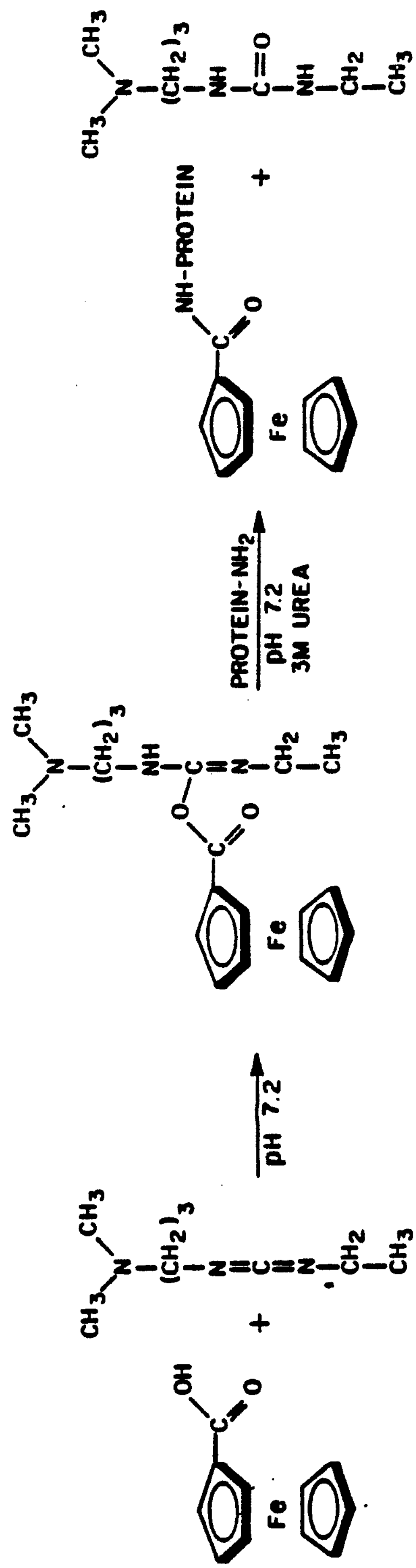


I II III IV V

This reaction sequence is utilized since the pH in the reaction medium does not adversely affect the enzyme, since the reaction proceeds in an aqueous medium, and since reagents that do not deactivate the enzyme are employed to induce the reaction. However, reaction sequences are generally adaptable to the particular functional groups available on the enzyme and to the reactions typically undergone by such groups. Although a particular reaction mechanism has been chosen to produce the modified enxyme, the electrical and chemical activity does not depend on the particular reaction employed. Alternatively, it is possible to introduce a redox center by modifying a functional group of the enzyme. For example, glucose oxidase is modified by reacting some of its tyrosine to form 3-amino-4-hydroxyphenylalanine and/or 3,4-dihydroxyphenylalanine.

The exact mechanism for introducing the presence of redox center without deactivating the enxyme is not known. A contemplated mechanism is that the enzyme-modifying redox center is sufficiently removed from redox centers originally present in the enzyme, that it does not interact chemically, and, thus, does not cause deactivation. However, the distance required for deactivation is significantly smaller than the distance through which electrons are transferable. Thus, it is believed that the added redox center is sufficiently far from the chemical center to avoid deactivation and yet sufficiently close to allow electron transfer. In dimeric or tetrameric enzymes, it is possible to expose the inner protein, possibly in the proximity of the enzymes redox centers, by reagents such as urea. In this case, bonding of the redox couples to the exposed inner-protein surface is adequate and avoids excessive change in the conformations of the monomeric enzymes. Surface bonding also produces effective charge transfer.

Precise control of the position of the introduced redox center is, however, generally not possible and thus some activity is lost. For example, in the modification of glucose by a ferrocene derivative, the enzyme's overall activity is reduced by one-third upon modifications. Nevertheless, despite such partial deactivation, it is possible to produce a sufficiently active enzyme that has the required chemical and electronic activity. Thus, the procedure for electronic modification is generally applicable to enzymes and the electrochemical procedures depending on them.

As discussed, it is possible to attach the enzyme to a substrate, e.g., an inert carrier, an electrode, or an electrode that is part of a semiconductor-based device. For example, it is possible to introduce a modified glucose oxidase into a porous polymer coated onto a platinum electrode. The advantage of such direct contact is that enzyme is not present in the medium and, thus, a smaller amount of enzyme is sufficient. However, such methods for contacting often reduce the efficiency of electron transfer between the modified enzyme and the electrode. For some applications where immunological interaction of the enzyme with the biological medium is not desired, use of both a membrane and electrode attachment is useful.

In the absence of direct attachment to the electrode, it is desirable to use a membrane to prevent diffusion of the enzyme to an excessive distance from the electrode in processes such as assays where such diffusion is unacceptable. Suitable membranes include materials such as porous glass or cellulose derivatives and biocompatible, unmodified, or modified materials including polyesters, polycarbonates, polyurethanes, methacrylates, polyvinyl alcohols, fluorinated polymers, e.g., Teflon®, and coated porous glasses.

The following examples are illustrative of the invention:

## Example 1

Glucose oxidase [E.C. 1.1.3.4] from Aspergillus niger was purchased from Sigma, St. Louis, Mo. (Catalog #G-8135). The activity of this enzyme was 118 units/mg. Na-HEPES[sodium 4-(2-hydroxyethyl)-1-piperzeineethanesulfonate], DEC[1-(3-dimethylamino propyl)-3-ethylcarboxiimide hydrochloride], and ferrocenecarboxylic acid (I), were purchased from Aldrich. SEPHADEX G-15 was purchased from Pharmacia Fine Chemicals. Catalase [E.C. 1.11.1.6] was purchased from Sigma (Catalog # C-10).

The enzyme was modified in the following way: All reactions were carried out in an ice bath. Approximately 80 mg of ferrocenecarboxylic acid was dissolved in 4 ml of a 0.15M Na-HEPES solution, to form a slightly turbid solution of pH 7.3 ± 0.1. (When higher, the pH was lowered to this value by adding dropwise, with stirring, an 0.1M HCl solution.) Approximately 100 mg of DEC was then added and dissolved, followed by 810 mg of urea. After the urea dissolved the pH was again adjusted to a value in the range 7.2 to 7.3 and 60 mg of glucose oxidase was added and dissolved. When necessary, the pH was again adjusted to the 7.2 to 7.3 range, by adding either 0.15 Na-HEPES or 0.1M HCl solution

The solution was then placed in a glass vial, sealed with a paraffin foil, and left immersed in an ice and water containing dewar overnight (~15h). The turbid solution was centrifuged, and the supernatant liquid

was filtered under mild (~2 atm) pressure through a 0.2 µm pore filter. The modified enzyme was separated from the unreacted ferrocenecarboxylate and from the reaction product of ferrocenecarboxylate and DEC by gel filtration through a 2 cm diameter, 22 cm long column of SEPHADEX G-15. Prior to the preparation of the column, the SEPHADEX was soaked for more than 2 hours in a pH 7.0 buffer, prepared by adding to a 0.085M solution of $Na_2HPO_4$ drops of concentrated HCl. This buffer was used as the eluent in the separation. The enzyme was eluted in the first, orange-colored fraction, of a volume of 4 ml or less. Analysis of solutions of the enzyme by atomic absorption spectroscopy showed that prior to modification there were 2 iron atoms per enzyme molecule, and that after modification there were 14 iron atoms per molecule.

The electrochemical activity of the enzyme was measured using an electrochemical cell that had two compartments. The inner, enzyme containing, compartment was a 1.0 cm ID pyrex tubing with a VF glass frit. The working electrode was polished sequentially with 1.0, 0.3, and 0.05 µm $Al_2O_3$, rinsed in deionized water, and dried in a nitrogen stream prior to each measurement. The reference electrode was NaCl-saturated calomel, and the counter electrode was spectroscopic graphite rod. The electrolyte in the outer compartment was the buffer used for gel-filtration. The measurements were performed under a nitrogen atmosphere, using a PAR 173 potentiostat/galvanostat with a PAR 175 programmer, and an HP 7090A recorder.

The cyclic voltammograms shown in Figure 1 were obtained for an enzyme concentration of 10 mg/ml (1200 units/ml in the case of the unmodified enzyme) in .085M $Na_2HPO_4$ brought to pH 7.0 by adding concentrated HCl. The solution also contained 6000 units/ml of catalase to decompose any hydrogen peroxide that might be formed in the presence of traces of oxygen.

Curve 30 of Figure 4, shows cyclic voltammograms of solutions of the enzyme before modification with and without 5.0 mM glucose. The cyclic voltammograms observed under identical conditions with the modified enzyme are seen in curves 31 (no glucose), 32 (0.8 mM glucose) and 33 (5.0 mM glucose) of Figure 4. While the unmodified enzyme did not respond to the addition of glucose, the modified enzyme did. The point at which half the glucose-concentration-dependent, limiting current is reached was 0.5V (SHE), which is consistent with the redox potential of the ferrocenecarboxylate/ferrociniumcarboxylic couple.

## Example 2

The enzyme D-amino acid oxidase [E.C. 1.4.3.3.] from porcine kidney (type I) was purchased from Sigma, St. Lous, Mo.. The enzyme was modified by ferrocenecarboxylic acid, and purified following the procedure described in Example 1. Cyclic voltametry for the unmodified enzyme yielded curve 40 (Figure 1). No increase in the oxidation current was observed when D-alanine was added to a solution of this non-modified enzyme. Cyclic voltametry for the modified enzyme showed an increasing oxidation current from curve 41 to curve 42 after 2mM D-alanine was added to the solution of the modified enzyme (Figure 1).

## Example 3

Ferrocenylacetic acid was prepared as described by D. Ledincer, J. K. Lindsay, and C. R. Hauser in the Journal of Organic Chemistry, 23, p. 653 (1958). The enzyme glucose oxidase [E.C. 1.1.3.4] was modified through the procedure described in Example 1, except that ferrocenylacetic acid was used instead of ferrocenecarboxylic acid. Figure 2 shows the change in oxidation current from curve 51 to curve 52 upon the addition of sufficient glucose to an electrochemical cell containing the modified enzyme to yield a 30 mM solution. A similar cell with the non-modified enzyme showed no change in current upon the addition of glucose.

Example 4

Tyrosine groups of glucose oxidase [E.C. 1.1.3.4] were converted to electrochemically active 3-amino-4-hydroxy-phenylalanine and DOPA groups through the following scheme:

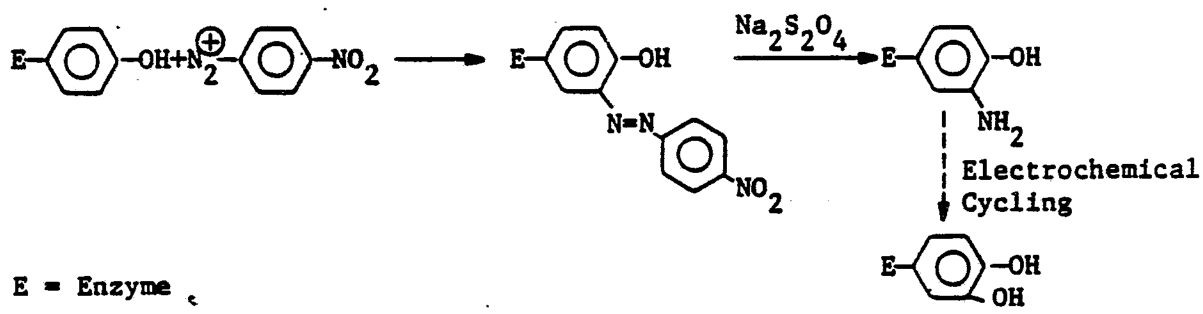


E = Enzyme

Approximately 140 mg of p-aminoitrobenzene was dissolved in 10 ml of boiling 0.3 M HCl. The solution was cooled to ice bath temperature and 70 mg of $NaNO_2$, dissolved in 1 ml of water, wad added. After stirring for 5 mins., 1 ml of this solution was mixed with 2 mg of a solution that 1) was 0.3 M in phosphate, buffered at pH 10, 2) was 2 M in urea, and 3) contained 60 mg of glucose oxidase [E.C. 1.1.3.4]. The resulting deep red solution was stirred for 10 mins., then subjected to gel-filtration, using a SEPHADEX G-15 column. The enzyme was eluted as the first deep red fraction. The azo-bond was reductively cleaved by adding $Na_2S_2O_4$ (50 mg) to 2 ml of the pH 7 solution of the modified enzyme. The mixture was allowed to react for 20 min. at room temperature and the enzyme fraction was separated by gel-filtration. The modified enzyme was eluted as the first orange fraction. The cyclic voltammograms obtained with the modified enzyme are shown in Figure 3. The curve 76 was obtained without glucose in the solution. The curve 83 was obtained using the same solution but also containing glucose at 30 mM concentration.

The oxidation curent increased when glucose was added to the solution of the modified enzyme.

Example 5

An electrode, on which the chemically-modified enzyme of Example 1 was immobilized was prepared using the immobilization technique of J. F. Castner and L. B. Wingard published in Biochemistry, 23, p. 2203, in 1984. In this procedure, a clean platinum-disc electrode 4 mm in diameter was placed in an electrochemical cell, containing 0.5 M sulfuric acid, and the electrode was cycled between 0.4 V (SCE) and 1.3 V (SCE) until the i-V characteristics stabilized. Subsequently, the electrode was reduced at -0.4 V (SCE) for 10 sec., then oxidized at +0.4 V (SCE) for 2 min. The electrode was then washed in deioized water and placed in an aqueous 4 (weight) percent allylamine solution for 30 min., rinsed in deionized water, and dreid in a nitrogen stream. Approximately 100 µl of the modified enzyme solution, prepared as described in Example 1, and 50 µl of a 2.5 (weight) percent glutaraldehyde solution in water were then mixed, and the platinum electrode on which an allylamine layer had been absorbed was contacted with this solution. The glutaraldehyde and the amine were allowed to react for 1 hour to form a polymer. The electrode was then rinsed with deionized water.

The glucose-response of this electrode was checked by measuring the current at a potential of 0.59V (SHE), e.g., 0.35V (SCE). The current measured when the electrolyte did not contain glucose was 100 µA. In the same electrolyte, but with 5 mM glucose in the solution, the current was 125 µA.

## Claims

1. An electrochemical process comprising the steps of interacting 1) a conductive aqueous-based medium containing a reactant with 2) an electrode, to induce a charge transfer and a reaction of said reactant wherein said transfer and said reaction involves an enzume characterized in that at least a portion of said charge transfer occurs directly between said electrode and said enzyme as a consequence of said reaction and wherein said reaction is accelerated by said enzyme.

2. The process of claim 1, characterized in that said charge transfer induces a desired chemical conversion.

3. The process of claim 1, characterised in that said enzyme comprises a material formed by the chemical reaction between an enzyme that is incapable of said direct exchange and a material having a first functional group that chemically reacts with said enzyme and a second functional group that is capable of acting as a redox center.

4. The process of claim 2, characterised in that said enzyme comprises an enzyme that is incapable of said direct transfer wherein said incapable enzyme has at least one of its functional groups modified into a redox functional group.

5. The process of claim 1, characterised in that said transfer exchange is monitored to determine the concentration or presence of a reactant.

6. The process of claim 5, characterised in that said enzyme comprises a material formed by the chemical reaction between an enzyme incapable of said direct transfer and a material having a first functional group that is capable of acting as a redox center and a second functional group that chemically reacts with said enzyme.

7. The process of claim 6, characterised in that said incapable enzyme is glucose oxidase.

8. The process of claim 7, characterised in that said material comprises a ferrocene derivative.

9. The process of claim 5, characterised in that said enzyme comprises an enzyme that is incapable of said direct transfer wherein said incapable enzyme has at least one of its functional groups modified into a redox functional group.

10. The process of claim 9, characterised in that said incapable enzyme is glucose oxidase.

11. The process of claim 1, characterised in that said enzyme is attached to said electrode.

12. The process of claim 1, characterised in that said interaction is done in the presence of a membrane.

13. The process of claim 12, characterised in that said membrane comprises a biocompatible membrane.

14. An apparatus suitable for electrochemical processes, said apparatus comprising an electrode and an enzyme characterized in that said enzyme is capable of direct charge transfer with said electrode as a consequence of a desired chemical reaction.

15. The apparatus of claim 14, characterised in that said enzyme is formed by reacting a) an enzyme incapable of direct charge transfer with said electrode with b) a material having a first functional group that chemical bonds with said enzyme and a second functional group that acts as a redox center.

16. The apparatus of claim 15, characterised in that said incapable enzyme comprises glucose oxidase.

17. The apparatus of claim 16, characterised in that said material comprises a ferrocene derivative.

18. The apparatus of claim 14, characterised in that said enzyme is formed by the modification into a redox center of a functional group of an enzyme incapable of undergoing direct charge transfer with said electrode.

FIG. 1

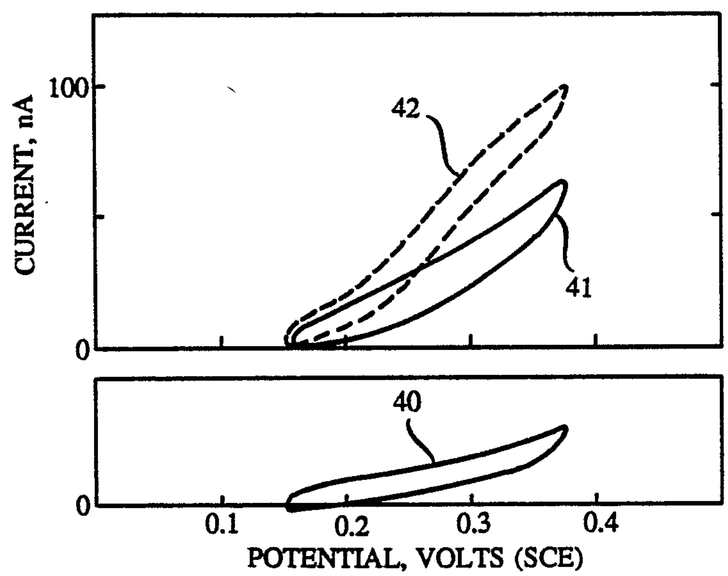
100
42
CURRENT, nA
41
0
40
0
0.1
0.2
0.3
0.4
POTENTIAL, VOLTS (SCE)

FIG. 2

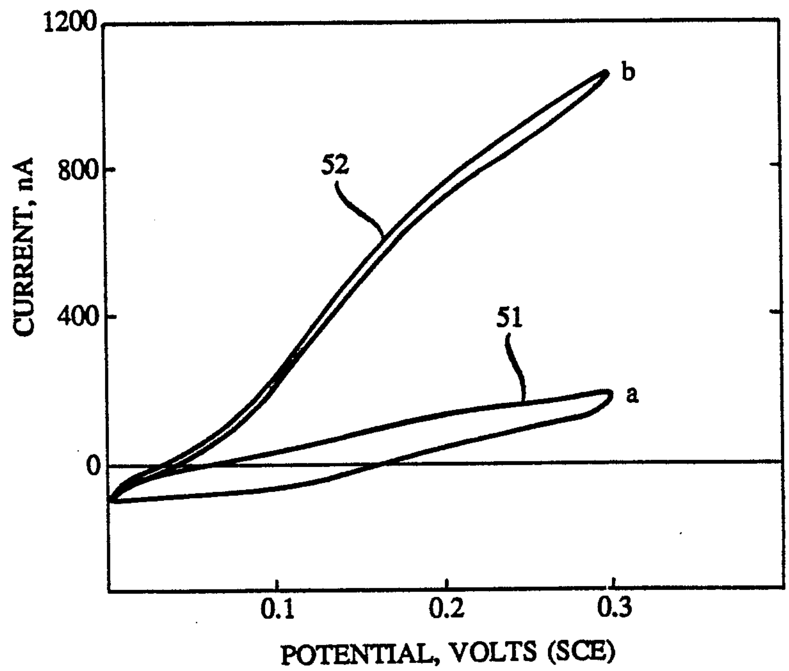
1200
b
800
52
CURRENT, nA
400
51
a
0
0.1
0.2
0.3
POTENTIAL, VOLTS (SCE)

FIG. 3

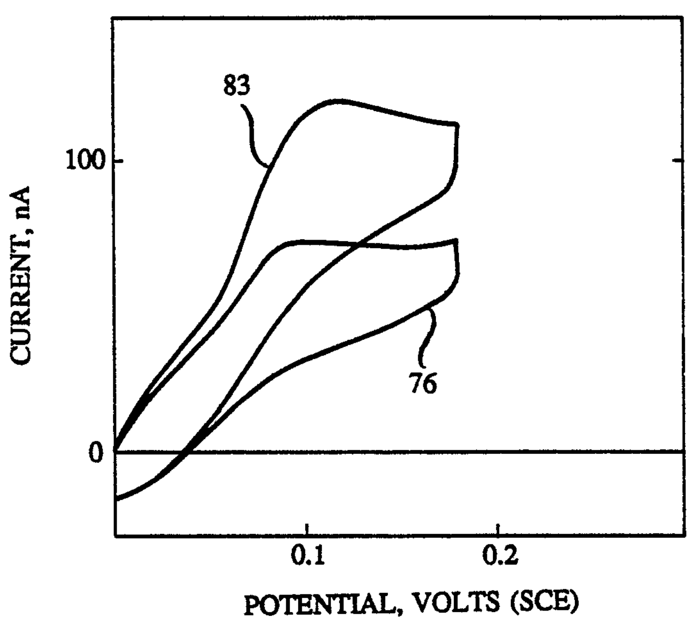

FIG. 4

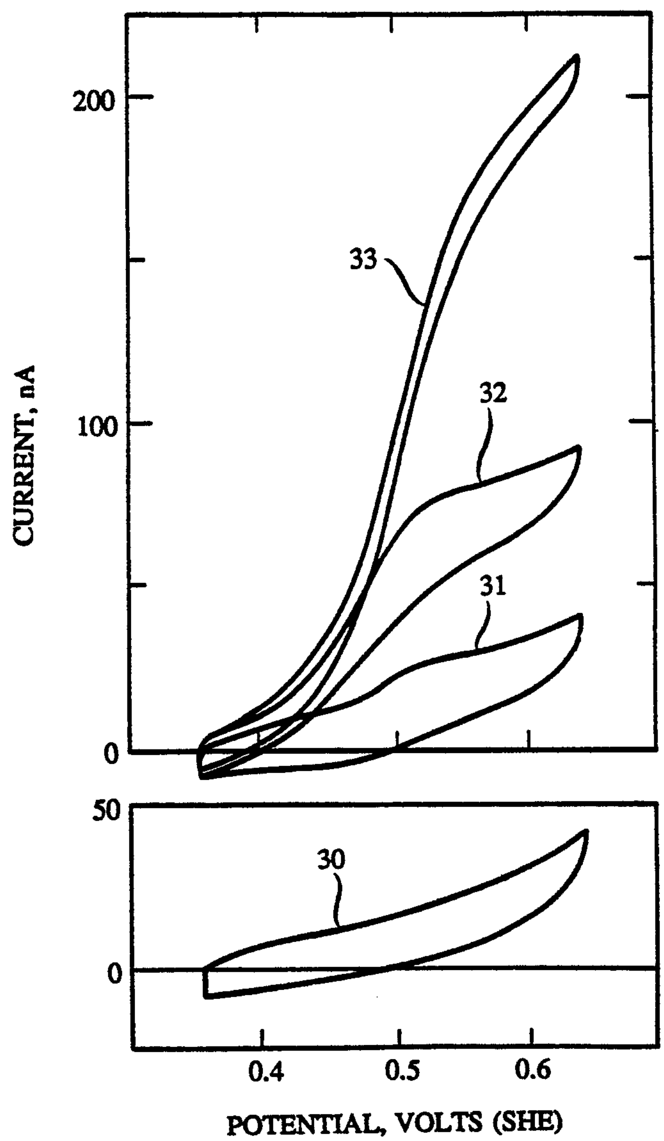
200
100
0
33
32
31
CURRENT, nA
50
0
30
0.4
0.5
0.6
POTENTIAL, VOLTS (SHE)